# EUROPEAN PATENT SPECIFICATION

(11) **EP 1 620 046 B1**
(45) Date of publication and mention of the grant of the patent: **12.10.2016**
(21) Application number: 04758808.2
(22) Date of filing: 01.04.2004
(51) Int. Cl.: A61F 2/30, A61F 2/50, A61F 2/78, A61F 2/80

(54) **SURE-FIT PROSTHETIC ANCHORING SYSTEM**
PROTHESTISCHES VERANKERUNGSSYSTEM MIT SICHEREM SITZ
SYSTEME D'ANCRAGE DE PROTHESE A FIXATION FIABLE

(30) Priority: 01.04.2003 US 405757
(43) Date of publication of application: 01.02.2006
(73) Proprietor: Mantelmacher, H., Lee, Owings Mills, MD 21117 (US)
(72) Inventor: Mantelmacher, H., Lee, Owings Mills, MD 21117 (US)
(74) Representative: Moffat, John Andrew
(86) International application number: PCT/US2004/010269
(87) International publication number: WO 2004/089261

(56) References cited:
- DE-A1- 10 026 399
- US-A- 4 923 474
- US-A- 5 211 667
- US-A- 5 651 792
- US-A- 5 653 766
- US-A1- 2002 077 705
- US-B1- 6 368 357
- US-B2- 6 666 894

## Description

### TECHNICAL FIELD

The present invention relates to prosthetics and, more particularly, to an anchoring system for post-operative prosthetic devices for above-the-knee amputation patients.

### BACKGROUND ART

There are a variety of different types of prosthetic devices for patients that have had either transfemoral (above-knee) or transtibial (below the knee) amputation. Typically, post-operative prosthetic devices for either type of amputation patients begin with a liner, which is rolled on to the residual limb. The liner is a soft, stretchy material that acts as an interface with between the skin and the socket of the prosthesis.

Once the liner is placed on the limb, the residual limb then slides into the hard socket of the prosthesis. This socket is custom made to fit the individual's residual limb and can be made out of a variety of materials.

Transfemoral prostheses have knee joints connected to them below the hard socket. The more fluid and natural the movement of the artificial knee the better the prosthesis. Transtibial prostheses have no knee joint. In both cases (with or without a knee joint) there typically is an aluminum or carbon fiber tube to which a foot module is connected.

For example, FIG. 1 is a prior art perspective view of the prosthetic device set forth in United States Patent No. 5,653,766 to Naser issued August 5, 1997. The illustrated prosthetic device 20 includes a generally cylindrical socket 24 having an opening at one end for receiving an amputated limb. The socket 24 is closed at the other end, and is mounted on a bendable knee joint. Once the limb is properly received within the socket 24, straps 38 are adjusted so that a secure fit is achieved. The patient then is able to walk using the prosthetic device 20.

With all transfemoral and/or transtibial prostheses (above & below the knee), it is very important that the socket be securely fitted to the limb and secured in place. Stability is a common problem as many existing anchoring systems use a single attachment point to hold the residual limb in place, and this typically leads to extraneous pivoting, rotation and shift during ambulation. Moreover, it is important to be able to easily adjust the anchoring system periodically inasmuch as the mass of the limb may change significantly over the course of a day. Consequently, there should be some means for adjustment on the fly. The above-referenced '766 patent uses a radial pressure-fit imposed by tightening two belts. However, this tends to squeeze the limb, adding to discomfort. Moreover, the radial pressure tends to pop the limb out of the socket over the course of a day.

The well-known ICEX® Socket System uses a lanyard kit as a docking and locking mechanism. The ICEX® Socket has a distal pin which docks with the prosthesis. A lanyard is connected to the liner through a slot in the bottom of the socket. The lanyard is pulled to allow the patient's residual limb, which is enclosed in the silicone liner, to be drawn into the socket by the lanyard. The lanyard is then anchored to the front of the socket. The lanyard has the advantage of allowing for adjustment of limb position within the socket. If the limb changes position because of volume change and/or the distal migration of the limb from the socket, the prosthesis can easily be adjusted by the lanyard to compensate: The lanyard method of donning the socket also significantly reduces pain directly related to the donning process with a pin-locking mechanism. However, it has been found that many amputees lack the room for, are unable to tolerate, or have difficulty engaging the distal pin. Others complain of pain associated with engagement of the pin.

Coyote Systems^{®} sells the Summit™ Lock for those who cannot tolerate the "pull" associated with distal pin lock and lanyard suspension methods. The Summit™ Lock eliminates the pin, and instead uses a ratchet-type ski buckle assembly (a strip with multiple lock teeth and an end tab), which is attached to the lateral aspect of the liner at about ischial level. The tab is fed through a hole in the socket and engages in the externally mounted lock. Positioning the tab through the socket wall controls the rotation. Mounting the lock externally makes it easy to engage and reduces jamming. While the Summit™ Lock is a more flexible design, it does not provide as secure a fit. The Summit™ Lock only holds the limb in place from the top of the socket and is prone to shift. In addition, the patient cannot tighten the fit of the liner in the socket from a sitting position but must put his/her full weight onto the prosthesis.

There remains a significant commercial need for a prosthetic anchoring system which provides a stable anchor for the limb liner with top-side and lower attachment which prevents all extraneous up and down motion, pivoting, rotation and shift during ambulation, and which allows a patient to easily tighten/readjust the fit of the limb in the socket from a sitting position.

### DISCLOSURE OF THE INVENTION

Accordingly, it is an object of the present invention to provide a prosthetic anchoring system which increases the stability of the liner anchor using a combination of top-side and lower attachments to create to anchor points in order to prevent all extraneous up and down motion, pivoting, rotation and shift.

It is another object to eliminate the need for distal pin locks, and yet allow a patient to easily anchor the liner in the socket, and to easily readjust/tighten the fit of the limb in the socket from a sitting position.

It is still another object to provide a prosthetic anchoring system which is simple, inexpensive and highly effective, and which allows extremely convenient on-the-fly adjustment of the limb/liner seating to improve the fit of the limb within the socket despite changes in the size of the limb over the course of a day.

In accordance with the foregoing object, the present device comprises an anchoring system for transfemoral and/or transtibial prostheses, comprising a liner for enveloping an amputee limb. The liner has an upper fastening strap and buckle suspended toward the upper end, and a corresponding lower fastening strap fixedly attached on the bottom end of the liner. The anchoring system also includes a containment socket for seating the liner. The containment socket has a pair of slots there through at positions corresponding to the upper strap and buckle and lower strap of the liner, respectively. To apply the anchoring system, the patient first applies the liner to his/her limb. The limb covered by the liner is then inserted into the socket with the lower strap and upper strap and buckle protruding out through the respective slots. The lower fastening strap is then threaded upwards and locked with the buckle of the upper strap.

The means of attaching the upper strap and buckle with the lower strap is of a type that allows the user easy one-handed release or tightening of the straps. For example, the buckle mechanism may include a simple looploc or D-ring, while the lower strap includes a standard Velcro® hook and loop attachment. The patient feeds the lower strap through the looploc and then pulls back down on the strap. This pulley action draws the limb/liner down into the socket until the limb/liner is securely seated in the socket. When fully seated, the lower fastening strap is secured to itself by the Velcro®. Alternatively, the buckle mechanism may be similar to a ladderloc.

The foregoing forms a suspension which holds the prosthesis on the limb. Moreover, inserting the fastening straps through slots absolutely prevents lateral shift as well as rotation. On the other hand, the patient need only readjust the easy adjustment buckle mechanism to adjust the position of the limb within the socket. Thus, if the limb changes position because of volume change and the distal migration of the limb into the socket, the prosthesis can easily be adjusted to compensate.

### BRIEF DESCRIPTION OF THE DRAWINGS

Other objects, features, and advantages of the present invention will become more apparent from the following detailed description of the preferred embodiment and certain modifications thereof, in which:

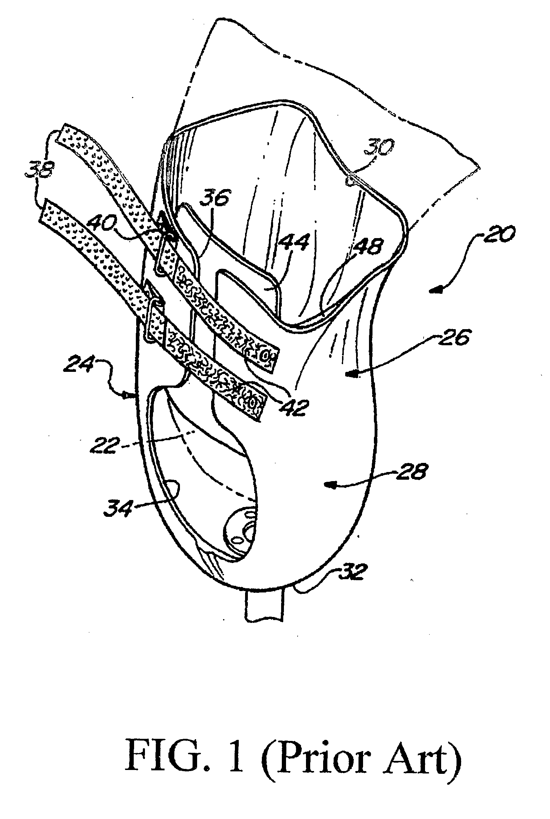

FIG. 1 is a prior art perspective view of the prosthetic device set forth in United States Patent No. 5,653,766 to Naser.
FIG. 2 is a perspective view of the components of the sure-fit prosthetic anchoring system 2 (including the liner 10, centering disk 33, and socket 12), according to the present invention, separated for purposes of illustration.
FIG. 3 is a perspective view of the liner 10.
FIG. 4 is a front view illustration of the liner 10 seated in socket 12.
5FIG. 5 is a composite perspective view 5A and top view 5B, respectively, of the cup insert 33.
FIG. 6 is a side view illustration of the liner 10 seated in socket 12.
FIG. 7 is a perspective illustration of an optional extension strap 44.
FIG. 8 is a perspective view of an alternative embodiment of the socket 120, modified with a slot 122 to avoid the need for centering cup 33.

### BEST MODE OF CARRYING OUT THE INVENTION

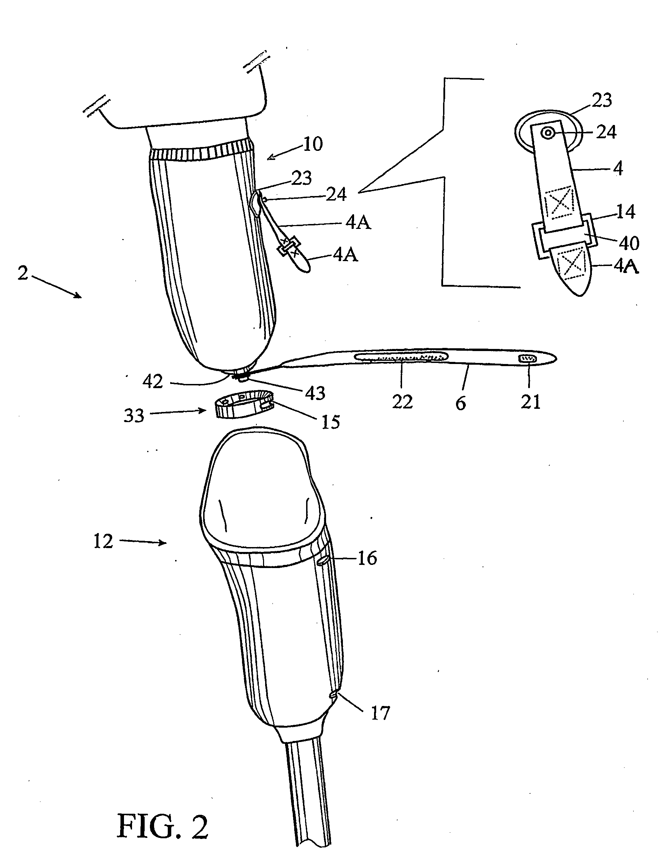

FIG. 2 is a perspective view of the sure-fit prosthetic anchoring system 2 according to one embodiment of the present invention. The primary components, including the liner 10,
centering disk 33 and socket 12, are separated for purposes of illustration. The anchoring system 2 is adapted for a patient that has undergone a limb reduction surgery resulting in an above-the-knee (transfemoral) amputation.

The anchoring system 2 generally includes a commercially-available liner 10 for covering the bony prominence of the above-knee residual limb (transfeinoral) or below-knee
(Trans-tibial) residual limb. As described more fully below, a variety of such liners are readily available and generally comprise a liner material enveloped by or impregnated into fabric. In accordance with the present invention the liner 10 is modified so as to be equipped with upper and lower fastening strap-anchors 4, 6, respectively. The upper strap anchor 4 is pivotally attached at one end by grommet and post component 24 to a reinforcement plate 23, plate 23
being a plastic member that is sewn and/or bonded peripherally onto the liner 10 at an upper outside position as shown. Presently, the grommet and post component 24 comprises two screw-together sections having I inch (2.5cm) flanges which sandwich the liner 10, plate 23 and upper strap 4 together. The other end of upper strap 4 bears a buckle mechanism 14. This buckle 14 is preferably of a type that allows easy one-handed adjustment, release or tightening of straps. In addition the strap 4 is bears a finger grip 4a. The finger grip 4a may be comprised of a short length of extension strap or plastic attached to the distal end of the buckle 14 itself, a slightly longer length sewn to the upper strap 4, or an even longer length of strap/plastic attached by the grommet and post component 24 directly.

In addition, a lower strap 6 is attached at one end to the bottom of the liner 10. The liner 10 fits within a molded socket 12 and rests within the socket 12 upon a centering cup 33. The socket 12 is formed with at least one slot 16 passing through an upper side (at the outside of the limb) for allowing the upper strap 4 and buckle 14 to pass outwardly therefrom. The centering cup 33 is formed with a slot 15 and the socket 12 is also formed with one lower slot 17 at the bottom. Slots 15 and 17 are horizontally aligned with each other and vertically aligned with the upper slot 16 for allowing the lower strap 6 to pass outwardly there through.

In the preferred embodiment, the lower fastening strap 6 has a section 21 of hook-and-loop (i.e. Velcro®) material at the distal end, and a mating section 22 of hook-and-loop material (i.e. Velcro®) running lengthwise along its mid-section. In addition the buckle 14 may be a conventional looploc (rectangular) or D-ring type buckle with a single aperture 40. In use, the patient would first apply liner 10 to limb. The liner 10 is then inserted into the socket with lower fastening strap 6 threaded through slot 15 of centering cup 33 out through lower slot 17, and upper strap 4 passing out through upper slot 16 with buckle 14 attached externally thereto. The fastening strap 6 is then threaded up through the aperture 40 of protruding buckle 14 and back downward. The strap 6 is pulled tight downward until the liner 10 is securely seated in the socket 12 atop cup 33. The fastening strap 6 is secured onto itself by joining the sections 21, 22 of hook-and-loop material. The foregoing forms a suspension which holds the prosthesis on and absolutely prevents lateral movement, pivotal shifting, and rotation. On the other hand, the simple Velcro®-attached strap 6 allows for convenient adjustment of the position of the limb within the socket 12. Thus, if the limb changes position because of volume change and the distal migration of the limb into the socket, the prosthesis can easily be adjusted by adjusting straps 4, 6 to compensate.

One skilled in the art will understand that buckle 14 may alternatively be a conventional ladder-lock buckle (having two or more apertures), or a cam-type buckle, without departing from the scope and spirit of the present invention. In these latter cases the Velcro self-attachment of the straps 4, 6 becomes unnecessary.

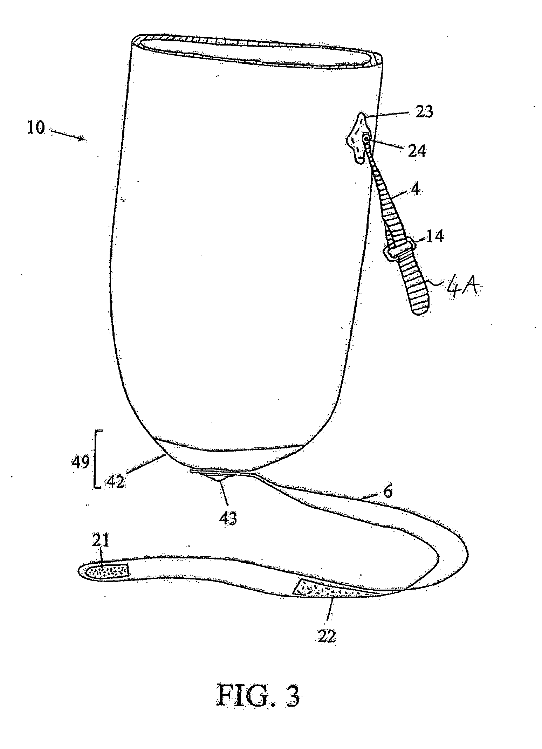

The particular components of the sure-fit anchoring system 2 will now be described in more detail with reference to FIGs. 3-6.

FIG. 3 is a perspective view of the liner 10. As stated previously, liner 10 is largely a standard transfemoral or transtibial suspension liner designed for amputees with amputations along the length of the femur or tibia, respectively. There are a variety of commercially-available suspension liners that will suffice, provided that they afford good suspension independent of volume fluctuations and provide a comfortable anatomical fit. These liners are typically formed of a liner material such as urethane, thermoplastic elastomer, silicone A, or silicone B, in thicknesses varying from about 6.94 mm to 9.17 mm, enveloped (or impregnated into) a fabric shell, and equipped with a threaded socket assembly 49 at the bottom end (typically intended for screw-insertion of a pin such as utilized in prior art pin securing assemblies). In accordance with the present invention, the otherwise conventional liner 10 is modified by tethering buckle 14, via upper strap 4, on the outwardly facing side of the liner 10. Strap 4 is secured to the liner 10 by first sewing and/or gluing the reinforcement pad 23 peripherally to the shell of the liner 10, and then passing a grommet-post 24 through the tip of the upper strap 4 and through the paid 23, thereby pivotally anchoring strap 4 thereto. Strap 4 is a short length (approximately 6 inches, 15cm) of braided Nylon or Dacron strap that is looped around one side of a buckle 14, thereby suspending buckle 14 approximately 3-5 inches (7.5-12.5cm) downward from the grommet-post 24. The buckle 14 is a simple rectangular stirrup-type stainless fixture (i.e. looploc) or alternately a D-ring, either with a single aperture 40. It should be understood that alternate embodiments are possible without departing from the scope and spirit of the invention, the point being that the tethered buckle 14 must be suspended by a short distance. Preferably, a short length (approximately 1-2 inches, 2.5-5cm) of strap material is attached to the opposing side of buckle 14 in a like manner and extends therefrom to provide a finger-grip 4a to facilitate insertion of the strap 4 and buckle 14 through the upper slot 16 in socket 12.

In addition to the upper strap 4 with buckle 14, the liner 10 is equipped with a lower fastening strap 6 comprising approximately a 2 feet length (60cm) of Nylon® or Dacron® braided strap attached at one end to the bottom of liner 10. As stated previously, the preferred liner 10 is equipped with a threaded socket assembly 49 (See FIG. 3) at the bottom end which includes a threaded metal screw-socket embedded in a concave rubber reinforcing pad 42 which is then epoxied and/or sewn, or otherwise fixedly attached to the distal end of the liner 10. A screw 43 is passed through the strap 6 and is screwed into the threaded socket assembly 49 to pivotally anchor the strap 4 thereto. In the preferred embodiment, the lower fastening strap 6 has a section 21 of hook-and-loop material (i.e. Velcro®) attached to the distal end, and a mating section 22 of hook-and-loop material running lengthwise along its mid-section for attaching strap 6 onto itself around buckle 14.

As described above, the preferred embodiment of the present invention includes a means for removably and adjustably attaching upper strap 4 and lower strap 6. In summary, this attachment means is comprised of strap 6 (with mating sections of Velcro® hook and loop material on the distal and medial sections) looped through a buckle mechanism 14 on strap 4, such as looploc type buckle component, and mated back unto itself. However, those skilled in the art will recognize that other attachment means may be used if they allow for easy one-handed release or tightening of the straps 4, 6. For example, a ladderloc type buckle mechanism may be used. While this ladderloc type of buckle may be more cumbersome initially because back threading of strap 6 through the buckle is required, the method of adjusting (pulling down on the strap 6) is the same.

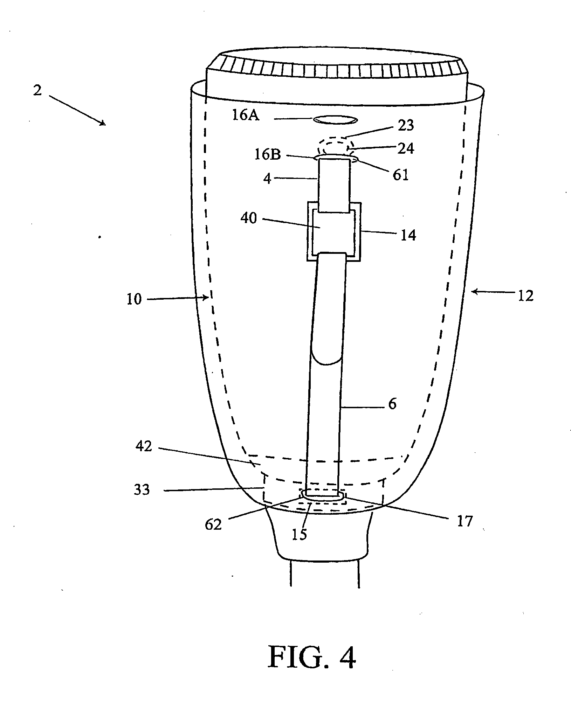

FIG. 4 is a perspective view of the liner 10 seated in socket 12 with the buckle mechanism 14 engaged connecting straps 4 and 6. Socket 12 is generally a conventional socket formed of flexible plastic that is vacuum formed. The socket 12 is a custom-fitted component made in a conventional manner using copolymer plastic, plastic polypropylene, polyester, acrylic/epoxy resin, etc. The socket 12 may be vacuum formed or thermoformed by heating the plastic material and forming it over a mold. In accordance with the present invention the socket 12 is formed with at least one pass-through slot 16 upwardly along the outside surface of the socket 12 corresponding to the outer surface of the leg. At least one pass-through slot 16 is required, although two or more facilitate adjustment. Thus, in place of the single slot shown in FIG. 2, a parallel pair of pass-through slots 16A & 16B (or even a larger series) may be used as shown in FIG. 4, the pair being positioned upwardly along the opposing sides of the socket 12. Whether a single slot 16, a pair 16A & 16B, or a series, the slots are spaced with respect to the liner 10 inserted therein so that they are aligned with the upper strap 4. Specifically, when the liner 10 is fully inserted at least one pass-through slot 16 should be even with the grommet-post 24 on liner 10. Other pass-through slots 16B, C... may be positioned slightly above or below for adjustment. This allows the tethered buckle 14 to be inserted directly through a selected slot 16A, 16B (as appropriate) from inside the socket 12 to outside, such that downward tension on strap 4 anchors the grommet-post 24 directly against the slot creating the first anchor point 61. In addition to the upper slot(s) 16 a lower pass-through slot 17 is positioned downwardly along the same outward side of the socket 12. The pass-through slot 17 is spaced with approximately a 2 inch (5cm) offset with respect to the bottom of the socket 12. This way, when the iner 10 is fully inserted the lower pass-through slot 17 allows the lower fastening strap 6 to be inserted there through and creates the second anchor point 62. These two anchor points 61. 62 provide the stability that prevents all extraneous up and down motion, pivoting, rotation and shift of the limb within the socket 12.

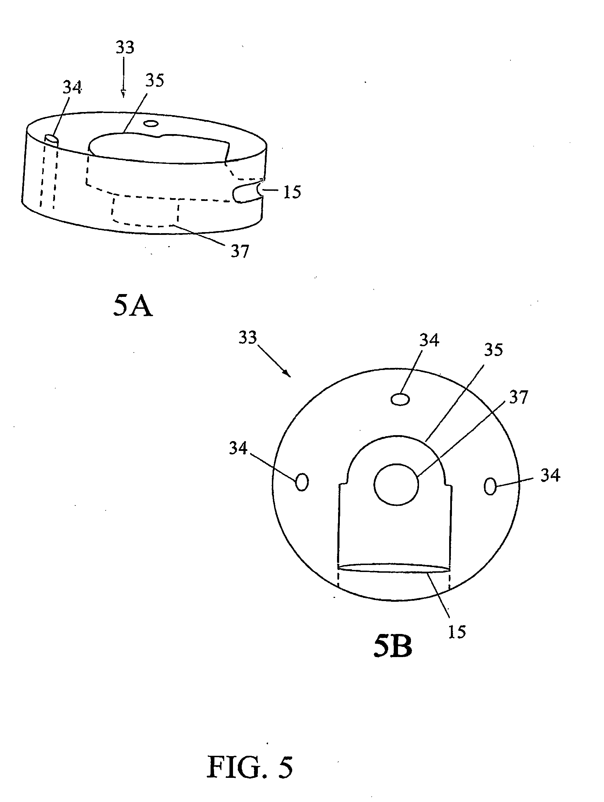

FIG. 5 is a composite perspective view 5A and top view 5B. respectively, of the centering cup insert 33 as in Fig. 2. While shown in FIG. 2 outside the socket 12, the centering cup 33 is attached internally to the bottom center of the socket 12. The centering cup 33 is a puck-like member preferably formed of Delrin®, aluminum, or other sturdy material. Centering cup 33 is formed with a partially concave upper surface leading into an alcove 35. The concave upper surface helps to seat and center the liner 10. The alcove 35 is semi-circular (on one side) with a pronounced indentation 37 in the center for seating the head of screw 43 on the liner 10. The alcove 35 continues through the other side of insert 33 forming a slot 15 through which the lower fastening strap 6 is passed. Through-bores 34 arc formed axially through the insert cup 33 to allow screw-attachment through the socket 12 into the base of a bendable knee joint (on a transfemoral prosthesis) or the base of the shaft (on a transtibial prosthesis), either of which are typically attached directly beneath the socket 12. Alternatively, the through-bores 34 may be eliminated and the centering cup 33 instead formed with a downwardly threaded hub for screw-attachment to the underlying base beneath socket 12.

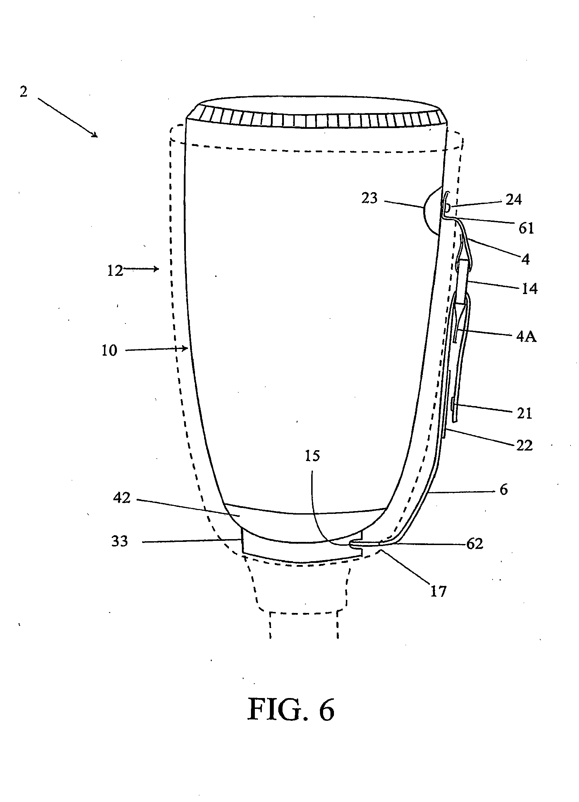

FIG. 6 is a side view of the liner 10 seated in socket 12 for purposes of illustrating the method of attaching the sure-fit prosthetic anchoring system 2 according to the present invention. To apply the anchoring system 2, the patient first applies the liner 10 to his/her residual limb. The liner 10 is then partially inserted into the socket 12 until lower fastening strap 6 can be threaded through the slot 15 in centering cup 33 and on outward through the lower slot 17 through socket 12. In addition, the upper fastening strap 4 and buckle 14 are passed outward through slot 16. The lower fastening strap 6 is then threaded up through the aperture 40 of buckle 14 (strap 6 running upward along the side of the socket 12) and is inserted there through. The patient then pulls down on the distal end of lower strap 6 which by pulley action draws the liner 10 down into the socket 12 until the liner 10 is securely seated in the socket 12. When fully seated, the lower fastening strap 6 is secured to itself by joining the sections 21, 22 of hook-and-loop material.

The foregoing forms a suspension which holds the prosthesis onto the limb. Moreover, the fastening strap 6 through lower slot 17 forms a first anchoring point for the liner 10 within the socket 12, and the upper strap 4 through upper slot 16 forms a second anchoring point, the combination of the two anchoring points 61, 62 serves to absolutely prevent lateral movement, pivotal and proximal shift, and rotation. On the other hand, the patient need only readjust the buckle mechanism 14 to adjust the position of the limb within the socket 12. Thus, if the limb changes position because of volume change and/or the distal migration of the limb into the socket 12, the prosthesis can easily be adjusted to compensate.

Once the limb is properly received within the socket 12 and the straps 4, 6 are appropriately adjusted to achieve a secure fit, the patient is able to walk using the prosthetic device.

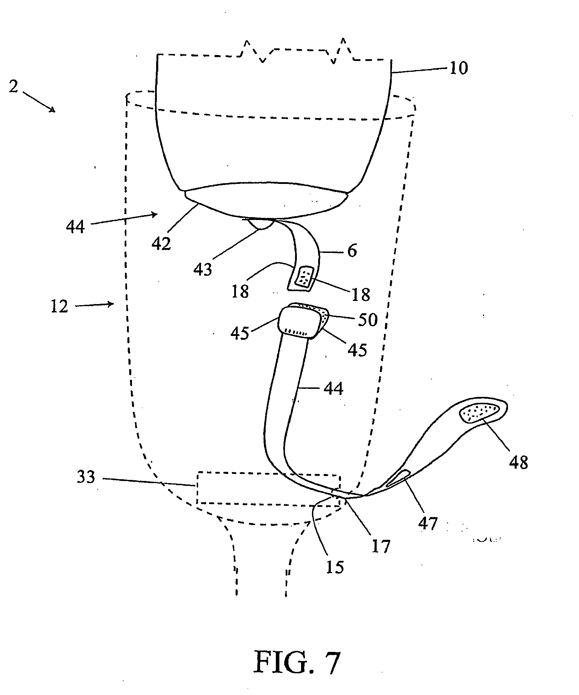

FIG. 7 is a perspective illustration of an optional extension strap 44, which can be used to simplify the attachment process for patients who lack upper-body mobility. Extension strap 44 essentially replaces the majority of the above-described, lower strap 6 but can be left in place in centering cup 33 to avoid having to thread strap 6 through cup 33 each time the prosthetic is applied. In this case, strap 6 is substantially shortened and a section of loop material 18 (i.e. Velcro® loop) is sewn to each face at the distal end. The extension strap 44 comprises a length of woven Nylon or Dacron (or similar material) belt with a clam-shell closure 45 at one end. The other end of extension strap 44 substantially conforms to that of lower strap 6, with mating sections of hook and loop material 47, 48 attached thereto on the distal and medial sections respectively. The clam-shell closure 45 further comprises two small plastic or leather patches attached at the end of extension strap 44 and able to close upon each other. The closure patches 45 at the end of extension strap 44 are equipped with opposing sections of hook material 50 (i.e. Velcro® hook) on their inner faces for a sandwich-type attachment to the loop material 18 at the end of shortened strap 6. In use, the patient again applies the liner 10 to his/her residual limb. The liner 10 is then partially inserted into the socket 12. The extension strap 44 is already inserted through the centering disk 33, outward through slot 15 of the centering disk 33, and slot 17 of the socket 12 as shown. Next, the patient closes the hook material 50 of clamshell closure 45 of extension strap 44 upon the loop 18 of strap 6, essentially forming an elongate strap which is pre- threaded and avoids excessive bending over. Just as before, the upper fastening strap 4 and buckle 14 are passed outward through slot 16 in socket 12. The extension strap 44 is then threaded up through aperture 40 of the buckle 14 (strap 44 running upward along the side of the socket 12). The patient pulls down on the distal end of extension strap 44 which works by pulley action to draw the liner 10 down into the socket 12 until the liner 10 (and thus the limb) is securely seated in the socket 12. When fully seated, the extension strap 44 is secured to itself by joining the sections 47, 48 of hook-and-loop material.

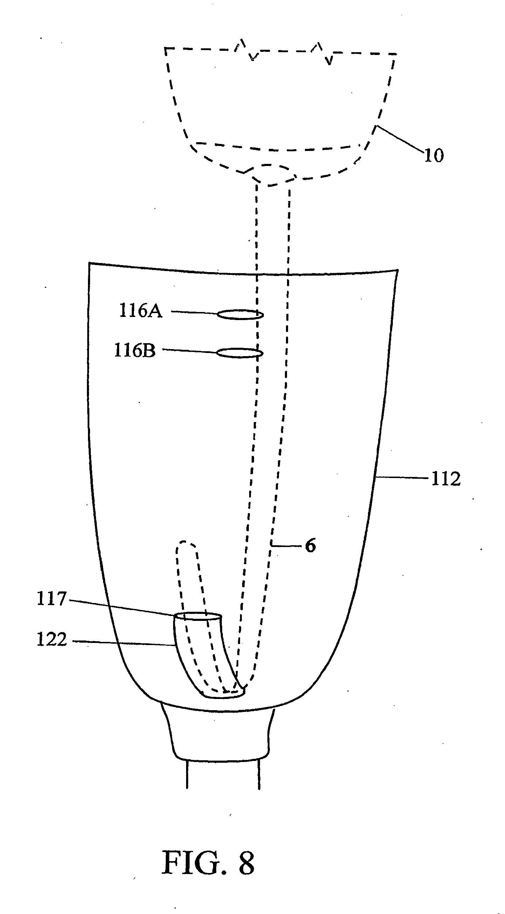

FIG. 8 is a perspective view of an alternative embodiment of the socket 112 similar yet modified slightly with a channel 122 opening onto slot 117. This channel 122 avoids the need for a centering cup 33. For example, if the prosthetic anchoring system is to be employed without a centering cup 33 using a liner 10 (as shown and described with reference to FIG. 3), the socket 12, as described above, would not provide enough clearance to be able to thread the lower strap 6 outward. To overcome this lack of clearance, the socket 112 must be thermoformed with a clearance channel 122. The clearance channel 122 is a raised channel for seating strap 6, and channel 122 runs to the clearance slot 117 which is (as before) located upwardly along the outside.

### INDUSTRIAL APPLICABILITY

In conventional transfemoral and transtibial prosthesis, a distal pin is used to engage and anchor a liner (in place on a residual limb) to the socket and a lanyard is used for adjusting the position of the limb. However, due these distal pins are often difficult for a patient to engage, especially when their injures impair flexibility. Other anchoring systems eliminate the need for distal pin locks but do not allow for easy adjustments from the sitting position as necessary if the limb changes position because of volume change and/or the distal migration of the limb into the socket. The prosthetic anchoring system of the present invention eliminates the need for distal pin locks, using combined top-side and lower anchors to prevent all extraneous up and down motion, pivotal and proximal shift, and rotation of the limb within the socket. It further allows the patient easy one-handed readjusting/tightening of the fit of the liner, and thus the limb within the socket, from a convenient sitting position.

Having now fully set forth the preferred embodiments and certain modifications of the concept underlying the present invention, various other embodiments as well as certain variations and modifications of the embodiments herein shown and described will obviously occur to those skilled in the art upon becoming familiar with said underlying concept.

## Claims

1. In combination with a soft liner (10) for enveloping an amputee limb, a socket (12) for receiving said amputee limb and liner, and a prosthesis attached to said socket, an anchoring system for anchoring said amputee limb and liner in said socket, **characterized by**:
an upper strap (4) secured at one end to an upper end of said liner;
a lower strap (6) secured at one end to a lower end of said liner;
a buckle (14) attached to the other end of one of said straps for removably receiving the looped-through other end of the other of said straps and thereby adjustably connecting said upper and lower straps;
an upper slot (16) through said socket for passing said upper strap, and a lower slot (17) through said socket for passing said lower strap;
whereby said upper and lower straps are inserted through said corresponding upper and lower slots when said liner is seated in said socket and are adjustably connected together by said buckle to secure said liner in said socket by a two-point-anchored suspension fit that prevents lateral, pivotal and proximal shift.

2. The anchoring system according to claim 1, further comprising a centering cup (33) seated in said socket and for seating said liner thereon.

3. The anchoring system according to claim 2, wherein said centering cup comprises a puck-shaped member (33) screw attached to the center bottom inside surface of said socket and having a concave upper surface to center the liner within the socket.

4. The anchoring system according to claim 3, wherein said centering cup is formed with a sidelong slot (15) to pass said lower strap (6) through to the lower slot (17) of the socket.

5. The anchoring system according to claim 4, wherein said buckle (14) allows for easy one-handed adjustment and tightening of said straps (4,6) and thereby adjustment of the position of said limb (enveloped in said liner) within the socket.

6. The anchoring system according to claim 5, wherein said buckle (14) is comprised of a single-aperture buckle consisting of a looploc or D-ring type buckle, and said lower strap (6) is further comprised of two mating sections (21,22) of hook and loop material sewn into distal and medial portions of said lower strap; whereby said upper and lower straps are connected by threading said lower strap through said aperture (40) of said buckle and securing the lower strap onto itself by said hook and loop material sections.

7. The anchoring system according to claim 5, wherein said buckle (14) is comprised of a multi-aperture ladder-lock buckle.

8. The anchoring system according to claim 5, wherein said buckle (14) is comprised of a cam-type buckle.

9. The anchoring system according to claim 1, wherein said lower strap further comprises a shortened first segment (6) attached to said liner and a second segment (44) pre-threaded through said centering cup (33) and lower slot (17) and removably attached to said first segment.

10. The anchoring system according to claim 1, wherein the socket (12) has a series of upper slots (16A, 16B; 116A,116B) and/or lower slots (17; 117) there through; said series of upper and lower slots corresponding approximately to the position of the upper and lower straps on the liner (10), respectively; whereby said series of slots allows for greater adjustment of said liner, and thereby said limb, within the socket.

11. The anchoring system according to claim 1. further comprised of a clearance channel (122) for seating and guiding the lower strap to the lower slot (117).

12. The anchoring system according to claim 1, further comprising a first reinforcing pad (23) sewn, glued, bonded or otherwise attached to the upper end of the liner (10) and a grommet and post (24) sandwiching said liner, reinforcement plate and upper strap (4) and thereby pivotally attaching said upper strap to said liner.

13. The anchoring system according to claim 1, wherein said upper and lower straps (4,6) are comprised of braided nylon or Dacron straps.

14. The anchoring system according to claim 13, wherein said upper strap is comprised of two segments connected on either side of a looploc buckle (14); the first segment (4) approximately 7.5-12.5cm in length and the second segment (4A) for use as a finger grip approximately 2.5-5cm in length; and wherein said lower strap is approximately 60cm in length.

15. The anchoring system according to claim 1, wherein said lower slot (17) is positioned approximately 5cm from the bottom of said socket.

16. The anchoring system according to claim 12, wherein said liner (10) is further comprised of a threaded socket assembly (49); said assembly including a threaded metal screw-socket embedded in a second reinforcing pad (42); said second reinforcing paid fixedly attached on the outer surface of the lower end of said liner; wherein said lower strap (6) includes a through bore at its proximal end; and wherein a screw is passed through through-bore of the lower strap and is screwed (43) into the threaded socket assembly to pivotally anchor the lower strap to the liner.

17. The anchoring system according to claim 1, for use with transfomoral and transtibial prostheses.

18. The anchoring system according to claim 1, wherein said buckled upper and lower straps (4,6) may be tightened and loosened from a seated position.

19. A method for anchoring an amputee limb in a prosthetic socket (12), comprising the steps of fitting a soft liner (10) over the amputee limb and inserting said limb with liner into the prosthetic socket;
**characterized by** said liner including an upper strap (4) connected thereto on one side and a lower strap (6) connected thereto on the same side, and a buckle (14) attached to one of said straps;
said socket having an upper slot (16) corresponding in position to said upper strap and a lower slot (17) corresponding in position to said lower strap;
inserting the upper and lower straps and buckle of the liner through the respective upper and lower slots of the socket;
adjustably connecting said upper and lower straps together externally of said socket by looping the other of said straps through said buckle;
adjusting a position of the limb within the socket by selectively tightening or loosening said upper and lower straps via said buckle.

## Patentansprüche

1. In Kombination mit einer weichen Einlage (10) zum Umschließen einer Gliedmaße eines Amputierten, Aufnahme (12) zum Aufnehmen der Gliedmaße des Amputierten und der Einlage, und eine mit der Aufnahme verbundene Prothese, ein Verankerungssystem zum Verankern der Gliedmaße des Amputierten und der Einlage in der Aufnahme, **gekennzeichnet durch**:
einen oberen Gurt (4), an einem Ende gesichert an einem oberen Ende der Einlage;
einen unteren Gurt (6), an einem Ende gesichert an einem unteren Ende der Einlage;
eine Schnalle (14), angebracht an dem anderen Ende eines der Gurte zum entfernbaren Aufnehmen des durchgeführten anderen Endes des anderen der Gurte und somit den oberen und den unteren Gurt einstellbar verbindend;
einen oberen Schlitz (16) **durch** die Aufnahme zum Durchführen des oberen Gurts und einen unteren Schlitz (17) **durch** die Aufnahme zum Durchführen des unteren Gurts;
wobei der obere und der untere Gurt **durch** den entsprechenden oberen und unteren Schlitz eingeführt werden, wenn die Einlage in der Aufnahme eingesetzt wird, und die mit der Schnalle einstellbar miteinander verbunden werden, um die Einlage in der Aufnahme über einen an zwei Punkten verankerten Tragesitz zu sichern, der laterales, drehendes und proximales Verlagern verhindert.

2. Verankerungssystem nach Anspruch 1, ferner umfassend eine Zentrierschale (33), eingesetzt in der Aufnahme und zum Einsetzen der Einlage darauf.

3. Verankerungssystem nach Anspruch 2, wobei die Zentrierschale ein puckförmiges Element (33) umfasst, das an die mittige untere Innenoberfläche der Aufnahme angeschraubt ist und eine konkave obere Oberfläche aufweist, um die Einlage in der Aufnahme zu zentrieren.

4. Verankerungssystem nach Anspruch 3, wobei die Zentrierschale mit einem seitlichen Schlitz (15) versehen ist, um den unteren Gurt (6) durch den unteren Schlitz (17) der Aufnahme zu führen.

5. Verankerungssystem nach Anspruch 4, wobei die Schnalle (14) ein einfaches einhändiges Einstellen und Festziehen der Gurte (4, 6) und somit ein Einstellen der Position der (von der Einlage umschlossenen) Gliedmaße in der Aufnahme ermöglicht.

6. Verankerungssystem nach Anspruch 5, wobei die Schnalle (14) eine Einzelöffnungsschnalle umfasst, die aus einer looploc- oder einer D-Ring-Schnalle besteht, und der untere Gurt (6) ferner zwei zusammenführbare Abschnitte (21, 22) aus Klettmaterial umfasst, die in einen distalen und einen medialen Abschnitt des unteren Gurts eingenäht sind; wobei der obere und der untere Gurt durch Führen des unteren Gurts durch die Öffnung (40) der Schnalle und Sichern des unteren Gurts an sich selbst durch die Klettmaterialbereiche verbunden sind.

7. Verankerungssystem nach Anspruch 5, wobei die Schnalle (14) eine Stegschnalle mit mehreren Öffnungen umfasst.

8. Verankerungssystem nach Anspruch 5, wobei die Schnalle (14) eine Nockenschnalle umfasst.

9. Verankerungssystem nach Anspruch 1, wobei der untere Gurt ferner Folgendes umfasst: ein verkürztes erstes Segment (6), das an der Einlage befestigt ist, und ein zweites Segment (44), das zuvor durch die Zentrierschale (33) und den unteren Schlitz (17) geführt ist und lösbar an dem ersten Segment befestigt ist.

10. Verankerungssystem nach Anspruch 1, wobei die Aufnahme (12) eine Reihe von durch diese verlaufenden oberen Schlitzen (16A,16B; 116A, 116B) und/oder unteren Schlitzen (17; 117) aufweist; wobei die Reihe von oberen und unteren Schlitzen nahezu der Position des oberen beziehungsweise unteren Gurts an der Einlage (10) entsprechen; wobei die Reihe von Schlitzen eine umfassendere Einstellung der Einlage und somit der Gliedmaße innerhalb der Aufnahme ermöglicht.

11. Verankerungssystem nach Anspruch 1, ferner einen Abstandkanal (122) zum Einsetzen und Führen des unteren Gurts hin zu dem unteren Schlitz (117) umfassend.

12. Verankerungssystem nach Anspruch 1, ferner umfassend ein erstes Verstärkungskissen (23), angenäht, geklebt, zusammengefügt oder auf andere Weise an dem oberen Ende der Einlage (10) befestigt, und eine Öse und einen Zapfen (24), die die Einlage, die Verstärkungsplatte und den oberen Gurt (4) einklemmen und somit den oberen Gurt drehbar an der Einlage befestigen.

13. Verankerungssystem nach Anspruch 1, wobei der obere und der untere Gurt (4, 6) verflochtene Nylon- oder Dacrongurte umfassen.

14. Verankerungssystem nach Anspruch 13, wobei der obere Gurt zwei Segmente umfasst, die auf beiden Seiten einer looploc-Schnalle (14) miteinander verbunden sind; wobei das erste Segment (4) etwa 7,5-12,5 cm lang ist und das zweite Segment (4A) für den Gebrauch als Fingergriff etwa 2,5-5 cm lang ist; und wobei der untere Gurt etwa 60 cm lang ist.

15. Verankerungssystem nach Anspruch 1, wobei der untere Schlitz (17) etwa 5 cm von dem Boden der Aufnahme entfernt angeordnet ist.

16. Verankerungssystem nach Anspruch 12, wobei die Einlage (10) ferner eine mit einem Gewinde versehene Muffenanordnung (49) umfasst; wobei die Anordnung eine in einem zweiten Verstärkungskissen (42) eingebettete mit Gewinde versehene Metallschraubmuffe enthält; wobei das zweite Verstärkungskissen fest an der Außenoberfläche des unteren Endes der Einlage befestigt ist; wobei der untere Gurt (6) eine durchgehende Bohrung an seinem proximalen Ende enthält; und wobei eine Schraube durch die durchgehende Bohrung des unteren Gurts geführt ist und in die mit einem Gewinde versehene Muffenanordnung geschraubt (43) ist, um den unteren Gurt drehbar an der Einlage zu verankern.

17. Verankerungssystem nach Anspruch 1 für den Gebrauch mit transfemoralen und transtibialen Prothesen.

18. Verankerungssystem nach Anspruch 1, wobei der obere und der untere Gurt (4, 6), mit der Schnalle befestigt, aus einer Sitzposition heraus festgezogen und gelöst werden können.

19. Verfahren zum Verankern einer Gliedmaße eines Amputierten in einer Prothesenaufnahme (12), umfassend die Schritte des Anpassens einer weichen Einlage (10) über die Gliedmaße des Amputierten und des Einführens der Gliedmaße mit der Einlage in die Prothesenaufnahme;
**dadurch gekennzeichnet, dass** die Einlage einen an einer Seite damit verbundenen oberen Gurt (4) und einen an derselben Seite damit verbundenen unteren Gurt (6) sowie eine an einem der Gurte befestigte Schnalle (14) enthält;
wobei die Aufnahme einen oberen Schlitz (16), dessen Position der des oberen Gurts entspricht, und eine unteren Schlitz (17), dessen Position der des unteren Gurts entspricht, aufweist;
Einführen des oberen und des unteren Gurts und der Schnalle der Einlage durch den entsprechenden oberen und den unteren Schlitz der Aufnahme;
einstellbares Verbinden des oberen und des unteren Gurts miteinander außerhalb der Aufnahme durch Führen des anderen der Gurte durch die Schnalle;
Einstellen einer Position der Gliedmaße innerhalb der Aufnahme durch selektives Festziehen oder Lösen des oberen und des unteren Gurts über die Schnalle.

## Revendications

1. En combinaison avec une doublure souple (10) permettant d'envelopper un membre d'amputé, une cavité (12) permettant de recevoir ledit membre d'amputé et ladite doublure, et une prothèse attachée à ladite cavité, système d'ancrage permettant d'ancrer ledit membre d'amputé et ladite doublure dans ladite cavité, **caractérisé par**:
une sangle supérieure (4) assujettie au niveau d'une extrémité à une extrémité supérieure de ladite doublure ;
une sangle inférieure (6) assujettie au niveau d'une extrémité à une extrémité inférieure de ladite doublure ;
une boucle (14) attachée à l'autre extrémité de l'une desdites sangles pour recevoir de manière amovible l'autre extrémité enveloppée de l'autre desdites sangles et
raccorder ainsi de manière ajustable lesdites sangles supérieure et inférieure ;
une fente supérieure (16) à travers ladite cavité permettant de faire passer ladite sangle supérieure, et une fente inférieure (17) à travers ladite cavité permettant de faire passer ladite sangle inférieure ;
moyennant quoi lesdites sangles supérieure et inférieure sont insérées à travers lesdites fentes supérieure et inférieure correspondantes lorsque ladite doublure est assise dans ladite cavité et sont raccordées de façon ajustable ensemble par ladite boucle pour assujettir ladite doublure dans ladite cavité par un ajustement de suspension ancré en deux points qui empêche un déport latéral, pivotant et proximal.

2. Système d'ancrage selon la revendication 1, comprenant en outre une coupelle de centrage (33) assise dans ladite cavité et permettant d'y asseoir ladite doublure.

3. Système d'ancrage selon la revendication 2, dans lequel ladite coupelle de centrage comprend une vis d'élément en forme de rondelle (33) attachée à la surface intérieure en bas au centre de ladite cavité et ayant une surface supérieure concave pour centrer la doublure au sein de la cavité.

4. Système d'ancrage selon la revendication 3, dans lequel ladite coupelle de centrage est formée avec une fente de côté (15) pour faire passer ladite sangle inférieure (6) à travers la fente inférieure (17) de la cavité.

5. Système d'ancrage selon la revendication 4, dans lequel ladite boucle (14) permet un ajustement et un serrage aisés à une main desdites sangles (4, 6) et ainsi un ajustement de la position dudit membre (enveloppé dans ladite doublure) au sein de la cavité.

6. Système d'ancrage selon la revendication 5, dans lequel ladite boucle (14) comprend une boucle monoouverture constituée d'une boucle de type Looploc ou anneau en D, et ladite sangle inférieure (6) comprend en outre deux sections d'accouplage (21, 22) de matériau autogrippant cousu dans des parties distale et médiale de ladite sangle inférieure ; moyennant quoi lesdites sangles supérieure et inférieure sont raccordées par filetage de ladite sangle inférieure à travers ladite ouverture (40) de ladite boucle et assujettissement de la sangle inférieure sur elle-même par lesdites sections dudit matériau autogrippant.

7. Système d'ancrage selon la revendication 5, dans lequel ladite boucle (14) comprend une boucle à double barrette autobloquante multiouverture.

8. Système d'ancrage selon la revendication 5, dans lequel ladite boucle (14) comprend une boucle de type came.

9. Système d'ancrage selon la revendication 1 dans lequel ladite sangle inférieure comprend en outre un premier segment raccourci (6) attaché à ladite doublure et un second segment (44) préfileté à travers ladite coupelle de centrage (33) et la fente inférieure (17) et attaché de manière amovible audit premier segment.

10. Système d'ancrage selon la revendication 1, dans lequel la cavité (12) comporte une série de fentes supérieures (16A, 16B; 116A, 116B) et/ou de fentes inférieures (17; 117) au travers ; ladite série de fentes supérieures et inférieures correspondant approximativement à la position des sangles supérieure et inférieure sur la doublure (10), respectivement ; moyennant quoi ladite série de fentes autorise un plus grand ajustement de ladite doublure, et ainsi dudit membre, au sein de la cavité.

11. Système d'ancrage selon la revendication 1, comprenant en outre un canal de débattement (122) permettant d'asseoir et de guider la sangle inférieure vers la fente inférieure (117).

12. Système d'ancrage selon la revendication 1, comprenant en outre un premier tampon de renforcement (23) cousu, collé, lié ou sinon attaché à l'extrémité supérieure de la doublure (10) et un oeillet et montant (24) prenant en sandwich ladite doublure, la plaque de renforcement et la sangle supérieure (4) et attachant ainsi de manière pivotante ladite sangle supérieure sur ladite doublure.

13. Système d'ancrage selon la revendication 1, dans lequel lesdites sangles supérieure et inférieure (4, 6) comprennent des sangles de nylon tressé ou de Dacron.

14. Système d'ancrage selon la revendication 13, dans lequel ladite sangle supérieure comprend deux segments raccordés de part et d'autre d'une boucle Looploc (14) ; le premier segment (4) ayant une longueur d'approximativement 7,5 à 12,5 cm et le second segment (4A) pour une utilisation comme crispateur ayant une longueur d'approximativement 2,5 à 5 cm ; et dans lequel ladite sangle inférieure a une longueur d'approximativement 60 cm.

15. Système d'ancrage selon la revendication 1, dans lequel ladite fente inférieure (17) est positionnée approximativement à 5 cm du fond de ladite cavité.

16. Système d'ancrage selon la revendication 12, dans lequel ladite doublure (10) comprend en outre un ensemble cavité filetée (49) ; ledit ensemble incluant une cavité de vis métallique filetée noyée dans un second tampon de renforcement (42); ledit second tampon de renforcement étant attaché fixement sur la surface externe de l'extrémité inférieure de ladite doublure ; dans lequel ladite sangle inférieure (6) inclut un alésage traversant au niveau de son extrémité proximale; et dans lequel une vis est passée à travers l'alésage traversant de la sangle inférieure et est vissée (43) dans l'ensemble cavité filetée pour ancrer de manière pivotante la sangle inférieure sur la doublure.

17. Système d'ancrage selon la revendication 1, pour une utilisation avec des prothèses transfémorale et transtibiale.

18. Système d'ancrage selon la revendication 1, dans lequel lesdites sangles supérieure et inférieure bouclées (4, 6) peuvent être serrées et desserrées depuis une position assise.

19. Procédé d'ancrage d'un membre d'amputé dans une cavité prothétique (12), comprenant les étapes d'ajustement d'une doublure souple (10) sur le membre d'amputé et d'insertion dudit membre avec la doublure dans la cavité prothétique; **caractérisé en ce que** ladite doublure inclut une sangle supérieure (4) qui lui est raccordée sur un côté et une sangle inférieure (6) qui lui est raccordée du même côté, et une boucle (14) attachée à l'une desdites sangles;
ladite cavité comportant une fente supérieure (16) correspondant en position à ladite sangle supérieure et une fente inférieure (17) correspondant en position à ladite sangle inférieure ;
d'insertion des sangles supérieure et inférieure et de la boucle de la doublure à travers les fentes supérieure et inférieure respectives de la cavité ;
de raccordement ajustable desdites sangles supérieure et inférieure conjointement de manière externe à ladite cavité par enveloppement de l'autre desdites sangles à travers ladite boucle;
d'ajustement d'une position du membre au sein de la cavité par serrage ou desserrage sélectif desdites sangles supérieure et inférieure via ladite boucle.
